(19) Europäisches Patentamt
European Patent Office
Office européen des brevets

(11) Veröffentlichungsnummer: **0 054 205**
**B1**

# (12) EUROPÄISCHE PATENTSCHRIFT

(45) Veröffentlichungstag der Patentschrift: **16.01.85**

(51) Int. Cl.⁴: **A 61 K 9/06,** A 61 K 9/10, A 61 K 31/415

(21) Anmeldenummer: **81109946.4**

(22) Anmeldetag: **27.11.81**

## (54) Antimykotische Mittel in Emulsionsform mit hoher Wirkstoff-Freisetzung.

(30) Priorität: **05.12.80 DE 3045913**

(43) Veröffentlichungstag der Anmeldung: **23.06.82 Patentblatt 82/25**

(45) Bekanntmachung des Hinweises auf die Patenterteilung: **16.01.85 Patentblatt 85/3**

(84) Benannte Vertragsstaaten: **AT BE CH DE FR GB IT LI NL SE**

(56) Entgegenhaltungen:
**DE - A - 2 461 406**
**FR - A - 2 275 194**
**US - A - 3 483 008**
**US - A - 3 502 594**

**E.J. ARIENS "Drug Design", Vol. IV, Academie Press (1978), p. 172**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **von Bittera, Miklos, Max-Scheler-Strasse 7, D-5090 Leverkusen 3 (DE)**
Erfinder: **Büchel, Karl Heinz, Prof. Dr., Dabringhausenerstrasse 42, D-5092 Burscheid (DE)**
Erfinder: **Plempel, Manfred, Dr., Pahlkestrasse 5, D-5600 Wuppertal 1 (DE)**
Erfinder: **Regel, Erik, Ing. grad., Bergerheide 72a, D-5600 Wuppertal 1 (DE)**

EP 0 054 205 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neuartige Formulierungen der bekannten antimykotischen Azolderivate, die eine höhere Freisetzung der Wirkstoffe aufweisen und dadurch eine Kurzzeittherapie ermöglichen.

Für die Behandlung von Mykosen beim Menschen, vor allem die Mykosen der Haut und der Hautanhangsgebilde sind bereits Zubereitungen von antimykotischen Derivaten bekannt geworden. Mit diesen Zubereitungen wurden für eine vollständige Sanierung 14 bis 21 Tage Therapiezeit benötigt.

Um z.B. bei Vaginal- oder Buccal-Mykosen oder Dermatomykosen zu einer Verkürzung der Therapiedauer zu kommen, benötigt man, besonders zur Eliminierung der Keime, bzw. um eine mykologisch gesicherte Sanierung zu erzielen, eine höhere Freisetzung der Wirkstoffe im wässrigen Milieu. Dafür sind die bekannten Formulierungen nur begrenzt geeignet, weil sich von dem vorhandenen Wirkstoffangebot nur ein kleiner Anteil im Flüssigkeitsvolumen am Ort der Infektion löst. Wenn man nun durch oder ohne weitere Erhöhung der Wirkstoffkonzentration eine Verkürzung der Therapiedauer, z.B. auf einen Tag bei einmaliger Applikation, erreichen will, muss man für eine optimale Bioverfügbarkeit des Wirkstoffes Sorge tragen.

Es wurde nun gefunden, dass solche Formulierungen antimykotischer Azolderivate in Emulsionsform, die neben üblichen Formulierungsstoffen 3-5% Benzylalkohol und 2,5-20% Spreitmittel enthalten, den Wirkstoff in höherem Masse freisetzen und dadurch eine Verkürzung der Therapiedauer auf einen Tag ermöglichen. Dieser Effekt der höheren Wirkstoff-Freisetzung kann bis zu einer Zehnerpotenz erreichen.

Wirkstoffe, die in dieser Weise formuliert werden können, sind alle antimykotisch wirksamen Azolderivate, insbesondere Imidazol- und Triazolderivate. Sie sind in den erfindungsgemässen Mitteln in Mengen von 0,05-1%, vorzugsweise 0,1-1%, vorhanden.

Beispielsweise seien als Wirkstoff die Verbindungen der nachstehenden Formeln genannt:

I 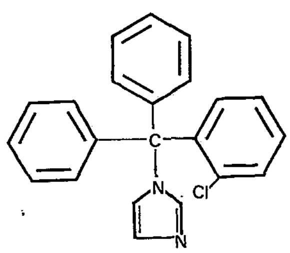 Clotrimazol (Bifonazol)

II 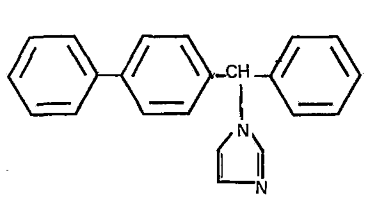 Trifonazol

III 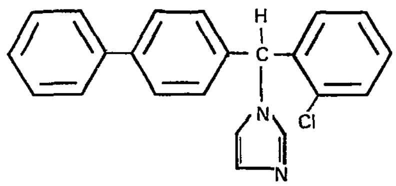 Lombazol

Zahlreiche weitere antimykotisch wirksame Azolderivate sind bekannt aus der DE-OS 2 430 039. Sie können ebenfalls in den erfindungsgemässen Mitteln als Wirkstoffe dienen.

Aus der DE-A-2 461 406 sind zwar schon Zubereitungen mit einem Gehalt an antimykotischen Azolderivaten, Benzylalkohol und Spreitmitteln bekannt. Aus DRUG DESIGN, E.J. Ariens, Volume IV, Academic Press, 1976, Seite 172, ist ausserdem bekannt, dass Benzylalkohol die Penetration von Arzneimitteln durch die Haut begünstigt. Bei den erfindungsgemässen Zubereitungen ist jedoch eine Penetration des Wirkstoffes durch die Haut durchaus unerwünscht, weil die Wirkung auf der Oberfläche der Haut stattfinden soll. Es war nun sehr überraschend, dass in den erfindungsgemässen Zubereitungen trotz der Gegenwart des Benzylalkohols praktisch keine Penetration stattfindet. Dieser Effekt wird erreicht durch die erfindungsgemässen Mengenverhältnisse, die durch die DE-A-2 461 406 keinesfalls offenbart oder nahegelegt werden.

Gemäss der Erfindung werden die folgenden Spreitmittel verwendet:

Isopropylmyristat, Isopropylpalmitat, Capryl/Caprinsäureester von gesättigten Fettalkoholen der Kettenlänge $C_{12}$-$C_{18}$, wachsartige Fettsäureester wie künstliches Entenbürzeldrüsenfett, Silikonöle, Isopropylmyristat/Isopropylstearat/Isopropylpalmitat-Gemisch, 2-Octyldodecanol, Ölsäuredecylester oder Koskosfettsäureisopropylester.

Folgende Hilfs- und/oder Formulierungs-Grundhilfsstoffe können bei der Herstellung der erfindungsgemässen Mittel verwendet werden.

| | |
|---|---|
| «Glycerylstearate», Gemisch von Mono- und Diglyceriden der Palmitin- und Stearinsäure | O/W Creme-Grundlage nicht selbst emulgierend |
| Kolloiddisperses Gemisch aus Cetyl-Stearylalkohol und Natrium-Cetyl-Stearylsulfat | O/W Emulsions-Grundlage selbst emulgierend |
| Ester einer verzweigten Fettsäure mit gesättigten Fettalkoholen $C_{16}$-$C_{18}$ | Ölkomponente mit ausgeprägt hydrophoben Effekt für Hautschutzpräparate |
| Cetylpalmitat | synthetischer Walrat, Konsistenz für Cremes, Salben, Emulsionen |
| Polyethylenstearat | nichtionogener hydrophyler Emulgator |

| | |
|---|---|
| Cetylstearylalkohol mit ca. 12 Mol Ethylenoxid | nichtionogener O/W Emulgator |
| Cetylstearylalkohol mit ca. 30 Mol Ethylenoxid | nichtionogener O/W Emulgator |
| Sorbitan und Glycerin-fettsäureester | nichtionogener Emulgator |
| Schwach vernetzte Polyacrylsäure von extrem hohem Molekulargewicht | Quellstoff, Verdikkungs- und Stabilisierungsmittel |

Weiterhin können auch folgende Formulierungshilfsstoffe verwendet werden:

Glycerin, Paraffin dickflüssig, Paraffin dünnflüssig, Triethanolamin, Collagen, Allantoin, Novantisolsäure, Parfümöle.

Ferner sind geeignet:

a. Substanzen, die z.B. eine Suspension stabilisieren können, z.B. kolloidale Kieselsäure, Montmorillonite u.a.
b. Tenside (beinhaltet Emulgatoren und Netzmittel), z.B.
   1. anionaktive, wie Na-Laurylsulfat, Fettalkoholethersulfate, Mono/Dialkylpolyglykolether-orthophosphorsäureester-Monoethanolaminsalz;
   2. kationaktive, wie Cetyltrimethylammoniumchlorid;
   3. ampholytische, wie Di-Na-N-lauryl-β-ininodipropionat oder Lecithin;
   4. nicht ionogene, z.B. polyoxyethyliertes Rizinusöl, polyoxyethyliertes Sorbitan-Monooleat, Sorbitan-Monostearat, Cetylalkohol. Glycerinmonostearat, Polyoxyethylenstearat, Alkylphenolpolyglykolether.
c. Stabilisatoren zur Verhinderung des bei einigen Wirkstoffen eintretenden chemischen Abbaues wie Antioxydantien, z.B. Tocopherole, Butylhydroxyanisol.
d. Weichmacher, z.B. Propylenglykol, Glycerin, Di- und tripropylenglykol, Triaethanolamin, Wachse.

Als Gelbildner kommen solche makromolekularen Verbindungen in Frage, die sich sowohl in Wasser als auch in organischen Lösungsmitteln lösen bzw. anquellen können und nach dem Trocknen eine Art Film bilden.

Folgt man einer Einteilung der makromolekularen Hilfsstoffe, [Keipert et al., Die Pharmazie 28, 145 bis 183 (1973)], so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Xanthan-Gummi, Guar-Gummi.

Amphotere Makromoleküle wie Protein-Derivate, z.B. Gelatine sind ebenso geeignet wie nichtionische Polymere, z.B. Methylcellulose, Hydroxypropylcellulose und lösliche Stärken, die obige Anforderungen erfüllen.

Als Lösungsmittel sind Wasser und auch alle mit Wasser mischbaren Lösungsmittel geeignet. In Betracht kommen z.B. Alkanole wie Ethanol und Isopropylalkohol, Propylenglykol, Methylcellosolve, Cellosolve, Ester, Morpholine, Dioxan, Dimethylsulfoxid, Dimethylformamid, Tetrahydrofuran, Cyclohexanon.

Es können bei der Herstellung der erfindungsgemässen Formulierungen ein oder mehrere Lösungsmittel eingesetzt werden.

*Beispiel 1* Emulsion, O/W

Phase I

| | |
|---|---|
| «Glycerylstearate» (Gemisch von Mono- und Diglyceriden der Palmitin- und Stearinsäure) | 8,00 g |
| 2-Octyldodecanol (Spreitmittel) | 10,00 g |
| Cetylstearylalkohol mit ca. 12 Mol Ethylenoxid | 1,50 g |
| Cetylstearylalkohol mit ca. 30 Mol Ethylenoxid | 1,50 g |
| Paraffin dickflüssig | 6,00g |
| 1,2-Propylenglykol | 5,00 g |
| Capryl/Caprinsäure-triglycerid | 6,00 g |

Die Mischung wird gerührt und bei 70°C geschmolzen.

Phase II

| | |
|---|---|
| Wasser entmineralisiert | 58,00 g |

Phase III

| | |
|---|---|
| Clotrimazol, | 1,00 g |
| gelöst in Benzylalkohol | 3,00 g |

Zur auf 75°C erwärmten Phase II wird die auf 70°C erwärmte Phase I unter Rühren hinzugefügt. Man lässt langsam auf 40°C abkühlen, gibt Phase III zu und lässt unter Rühren auf Raumtemperatur abkühlen. Die Rohemulsion wird bei 20 bis 25°C im Hochdruckhomogenisator homogenisiert.

In analoger Weise werden die Beispiele 2 und 3 verarbeitet.

*Beispiel 2* Emulsion, O/W

| | |
|---|---|
| Trifonazol micron. | 1,00 g |
| «Glycerylstearate», Gemisch von Mono- und Diglyceriden der Palmitin- und Stearinsäure | 9,00 g |
| Cetylstearylalkohol mit ca. 12 Mol Ethylenoxid | 3,00 g |
| 2-Octyldodecanol (Spreitmittel) | 10,00 g |
| Paraffin dickflüssig | 5,00 g |
| Benzylalkohol | 5,00 g |
| Wasser entmineralisiert | ad 100 ml |

*Beispiel 3* Emulsion, O/W

| | |
|---|---|
| Trifonazol micron. | 1,00 g |
| «Glycerylstearate», Gemisch von Mono- und Diglyceriden der Palmitin- und Stearinsäure | 9,00 g |
| Cetylstearylalkohol mit ca. 12 Mol Ethylenoxid | 3,00 g |
| 2-Octyldodecanol (Spreitmittel) | 10,00 g |
| Benzylalkohol | 5,00 g |
| Isopropylmyristat | 5,00 g |
| Wasser entmineralisiert | ad 100 ml |

*Beispiel 4* Creme, weiche Konsistenz O/W

| | | |
|---|---|---|
| Clotrimazol micron. | | 1,00 g |
| «Glycerylstearate», Gemisch von Mono- und Diglyceriden der Palmitin- und Stearinsäure | | 4,00 g |
| Cetylpalmitat | | 4,00 g |
| Cetylstearylalkohol mit ca. 12 Mol Ethylenoxid | | 1,00 g |
| Cetylstearylalkohol mit ca. 30 Mol Ethylenoxid | | 1,00 g |
| Isopropylmyristat/Isopropylpalminat, Isopropylstearat-Gemisch (Spreitmittel) | | 5,00 g |
| Schwach vernetzte Polyacrylsäure von extrem hohem Molekulargewicht | | 0,50 g |
| Natriumhydroxid 45%ig | | 0,11 g |
| Glycerin | | 3,00 g |
| Benzylalkohol | | 3,00 g |
| Wasser entmineralisiert | ad | 100 ml |

*Beispiel 5* Creme, weiche Konsistenz O/W

| | | |
|---|---|---|
| Clotrimazol micron. | | 1,00 g |
| «Glycerylstearate», Gemisch von Mono- und Diglyceriden der Plamitin- und Stearinsäure | | 4,00 g |
| Cetylpalmitat | | 4,00 g |
| Cetylstearylalkohol mit ca. 12 Mol Ethylenoxid | | 1,00 g |
| Cetylstearylalkohol mit car. 30 Mol Ethylenoxid | | 1,00 g |
| Isopropylmyristat/Isopropylpalmitat, Isopropylstearat-Gemisch (Spreitmittel) | | 5,00 g |
| Schwach vernetzte Polyacrylsäure von extrem hohem Molekulargewicht | | 0,50g |
| Natriumhydroxid 45%ig | | 0,11 g |
| Glycerin | | 3,00 g |
| Benzylalkohol | | 3,00 g |
| Wasser entmineralisiert | ad | 100 ml |

*Beispiel 6* Creme, weiche Konsistenz O/W

| | | |
|---|---|---|
| Trifonazol micron. | | 1,00 g |
| «Glycerylstearate», Gemisch von Mono- und Diglyceriden der Palmitin- und Stearinsäure | | 4,00 g |
| Cetylpalmitat | | 4,00 g |
| Cetylstearylalkohol mit ca. 12 Mol Ethylenoxid | | 1,00 g |
| Cetylstearylalkohol mit ca. 30 Mol Ethylenoxid | | 1,00 g |
| Isopropylmyristat/Isopropylpalmitat, Isopropylstearat-Gemisch (Spreitmittel) | | 5,00 g |
| Schwach vernetzte Polyacrylsäure von extrem hohem Molekulargewicht | | 0,50 g |
| Natriumhydroxid 45%ig | | 0,11 g |
| Glycerin | | 3,00 g |
| Benzylalkohol | | 3,00 g |
| Wasser entmineralisiert | ad | 100 ml |

*Beispiel 7* Emulsion, nichtfettend O/W

Phase I

| | |
|---|---|
| Ölsäuredecylester (Spreitmittel) | 2,50 g |
| Isopropylmyristat (Spreitmittel) | 2,50 g |
| Paraffin dünnflüssig | 4,00 g |
| Polyethylenstearat | 0,90 g |
| Sorbitan- und Glycerinfettsäureester | 0,60 g |

Die Mischung wird 10 Minuten bei 70°C gerührt und geschmolzen.

Phase II

| | |
|---|---|
| Wasser entmineralisiert | 50,00 g |
| Allantoin | 0,10 g |

*Carbopolschleim*

| | |
|---|---|
| Alkohol vergällt | 10,00 g |
| Carbopol 934 (schwach vernetzte Polyacrylsäure) | 0,70 g |
| Wasser entmineralisiert | 22,945 g |

Man dispergiert mit Turrax, lässt 2 Stunden quellen und neutralisiert dann mit 0,155 g 45%iger Natronlauge.

Zur 75°C heissen Phase II gibt man die 70°C warme Phase I unter Rühren hinzu und kühlt auf 45°C ab. Bei 45°C Carbopol-Schleim einrühren und bis 40°C weiter abkühlen. Bei 40°C 1,00 g Collagen zugeben und auf 25°C abkühlen. 1,0 g Lombazol in 3,0 g Benzylalkohol lösen und zur Phase I und II zugeben.

Anschliessend wird 0,600 g Parfum hinzugegeben und die Rohemulsion unter Rühren bei 20°C bis 25°C im Hochdruckhomogenisator homogenisiert.

In analoger Weise werden die Beispiele 8, 9, 10, 11 verarbeitet.

*Beispiel 8* Emulsion, nichtfettend O/W

| | | |
|---|---|---|
| Lombazol micron. | | 0,10 g |
| Ölsäuredecylester (Spreitmittel) | | 2,50 g |
| Isopropylmyristat (Spreitmittel) | | 2,50 g |
| Paraffin dünnflüssig | | 4,00 g |
| Polyethylenstearat | | 0,90 g |
| Sorbitan und Glycerinfettsäureester | | 0,60 g |
| Allantoin | | 0,10 g |
| Natriumhydroxid 45%ig | | 0,155 g |
| Schwach vernetzte Polyacrylsäure von extrem hohem Molekulargewicht | | 0,70 g |
| Collagen | | 1,00 g |
| Benzylalkohol | | 3,00 g |
| Ethanol | | 10,00 g |
| Parfümöl | | 0,60 g |
| Wasser entmineralisiert | ad | 100 ml |

*Beispiel 9* Emulsion, nichtfettend O/W

| | | |
|---|---|---|
| Lombazol micron. | | 0,05 g |
| Ölsäuredecylester (Spreitmittel) | | 2,50 g |
| Isopropylmyristat (Spreitmittel) | | 2,50 g |
| Paraffin dünnflüssig | | 4,00 g |
| Polyethylenstearat | | 0,90 g |
| Sorbitan- und Glycerinfettsäureester | | 0,60 g |
| Allantoin | | 0,10 g |
| Schwach vernetzte Polyacrylsäure von extrem hohem Molekulargewicht | | 0,70 g |
| Natriumhydroxid 45%ig | | 0,155 g |
| Collagen | | 1,00 g |
| Benzylalkohol | | 3,00 g |
| Ethanol | | 10,00 g |
| Parfümöl | | 0,60 g |
| Wasser entmineralisiert | ad | 100 ml |

*Beispiel 10* Emulsion, nichtfettend O/W

| | |
|---|---|
| Trifonazol micron. | 0,10 g |
| Ölsäuredecylester (Spreitmittel) | 2,50 g |

| | | |
|---|---|---|
| Isopropylmyristat (Spreitmittel) | | 2,50 g |
| Paraffin dünnflüssig | | 4,00 g |
| Polyethylenstearat | | 0,90 g |
| Sorbitan und Glycerinfettsäureester | | 0,60 g |
| Allantoin | | 0,10 g |
| Natriumhydroxid 45%ig | | 0,155 g |
| Schwach vernetzte Polyacrylsäure von extrem hohem Molekulargewicht | | 0,70 g |
| Collagen | | 1,00 g |
| Benzylalkohol | | 3,00 g |
| Ethanol | | 10,00 g |
| Parfümöl | | 0,60 g |
| Wasser entmineralisiert | ad | 100 ml |

*Beispiel 11* Emulsion, nichtfettend O/W

| | | |
|---|---|---|
| Trifonazol micron. | | 0,05 g |
| Ölsäuredecylester (Spreitmittel) | | 2,50 g |
| Isopropylmyristat (Spreitmittel) | | 2,50 g |
| Paraffin dünnflüssig | | 4,00 g |
| Polyethylenstearat | | 0,90 g |
| Sorbitan und Glycerinfettsäureester | | 0,60 g |
| Allantoin | | 0,10 g |
| Natriumhydroxid 45%ig | | 0,155 g |
| Schwach vernetzte Polyacrylsäure von extrem hohem Molekulargewicht | | 0,70 g |
| Collagen | | 1,00 g |
| Benzylalkohol | | 3,00 g |
| Ethanol | | 10,00 g |
| Parfümöl | | 0,60 g |
| Wasser entmineralisiert | ad | 100 ml |

*Beispiel 12* Creme, weiche Konsistenz O/W

| | | |
|---|---|---|
| Trifonazol micron. | | 0,10 g |
| «Glycerylstearate», Gemisch von Mono- und Diglyceriden der Palmitin- und Stearinsäure | | 4,00 g |
| Cetylpalmitat | | 4,00 g |
| Cetylstearylalkohol mit ca. 12 Mol Ethylenoxid | | 1,00 g |
| Cetylstearylalkohol mit ca. 30 Mol Ethylenoxid | | 1,00 g |
| Isopropylmyristat/Isopropylpalmitat, Isopropylstearat-Gemisch (Spreitmittel) | | 5,00 g |
| Schwach vernetzte Polyacrylsäure von extrem hohem Molekulargewicht | | 0,50 g |
| Natriumhydroxid 45%ig | | 0,11 g |
| Glycerin | | 3,00 g |
| Benzylalkohol | | 3,00 g |
| Wasser entmineralisiert | ad | 100 ml |

*Beispiel 13* Creme, weiche Konsistenz O/W

| | | |
|---|---|---|
| Trifonazol micron. | | 0,05 g |
| «Glycerylstearate», Gemisch von Mono- und Diglyceriden der Palmitin- und Stearinsäure | | 4,00 g |
| Cetylpalmitat | | 4,00 g |
| Cetylstearylalkohol mit ca. 12 Mol Ethylenoxid | | 1,00 g |
| Cetylstearylalkohol mit ca. 30 Mol Ethylenoxid | | 1,00 g |
| Isopropylmyristat/Isopropylpalmitat, Isopropylstearat-Gemisch (Spreitmittel) | | 5,00 g |
| Schwach vernetzte Polyacrylsäure von extrem hohem Molekulargewicht | | 0,50 g |
| Natriumhydroxid 45%ig | | 0,11 g |
| Glycerin | | 3,00 g |
| Benzylalkohol | | 3,00 g |
| Wasser entmineralisiert | ad | 100 ml |

*Beispiel 14* (Creme, weiche Konsistenz O/W)

| | | |
|---|---|---|
| Lombazol | | 1,00 g |
| «Glycerylstearate», Gemisch von Mono- und Diglyceriden der Palmitin- und Stearinsäure | | 4,00 g |
| Na-Stearat | | 16,00 g |
| Cetylstearylalkohol mit ca. 12 Mol Ethylenoxid | | 3,00 g |
| Benzylalkohol | | 3,50 g |
| 2-Octyldodecanol (Spreitmittel) | | 2,50 g |
| Kokosfettsäure-Isopropylester (Spreitmittel) | | 2,50 g |
| (Isopropylmyristat/Isopropylpalmitat/ Isopropylstearat-Gemisch) | | |
| Paraffin dünnflüssig | | 3,00 g |
| Wasser entmineralisiert | ad | 100,00 ml |

*Beispiel 15* (Creme transparent in Gelform)

| | | |
|---|---|---|
| Clotrimazol | | 1,00 g |
| Cetylstearylalkohol mit ca. 30 Mol Ethylenoxid | | 14,00 g |
| Kokosfettsäure-Isopropylester (Spreitmittel) | | 20,00 g |
| 2-Octyldodecanol (Spreitmittel) | | 5,00 g |
| Benzylalkohol | | 3,00 g |
| Wasser entmineralisiert | ad | 100,00 ml |

Wirksamkeit der erfindungsgemässen Mittel am Trichophyton-infizierten Meerschweinchen.

Als Testmodell zur vergleichenden Wirksamkeitsprüfung der erfindungsgemässen Formulierungen verwendeten wir Trichophyton-infizierte Pirbrightwhite-Meerschweinchen mit einem durchschnittlichen Gewicht von 600 g. Die Tiere wurde auf dem Rücken mit einer elektrischen Haarschneidmaschine so geschoren, dass ca. 1/10 mm lange Haarstümpfe stehen blieben.

Die Infektion mit Trichophyton mentagrophytes erfolgte durch leichtes Verreiben einer 24 Stunden in Sabouraud-Nährlösung angekeimten Sporensuspension des Erregers auf einer ca. 2 × 2 cm grossen Fläche des geschorenen Rückens der Tiere. Aufgetragen wurden pro Tier 0,5 ml Keimsuspension, die $1 - 3 \times 10^5$ infektiöse Pilzpartikel enthielten.

Bei diesem Infektionsmodus zeigen sich 2 - 3 Tage post infectionem die ersten Symptome der Dermatophytose als Rötung und Schuppung der Haut. Bei unbehandelten Tieren ist ca. 14 Tage p.i. die Dermatophytose maximal ausgeprägt. Flächiger Haarausfall und blutige Integument-Defekte innerhalb einer entzündlich veränderten, schuppigen Randzone.

Die zu prüfenden Formulierungen wurden 1mal, am 2. Tag post infectionem, lokal auf die gerötete Infektionsstelle der Tiere appliziert und mit einem Hornspatel leicht verrieben. Es wurden jeweils 0,5 ml der Formulierungen = 5 mg Wirkstoff (1%ige Formulierung) aufgetragen. Die Bewertung des Infektionsablaufs erfolgte täglich bis zum 20. Tag p.i.

Die Testergebnisse sind aus der nachstehenden Tabelle ersichtlich.

| Mittel des Beispiels | Wirkung am Trichophyton-infizierten Meerschweinchen |
|---|---|
| 1 | **** |
| 2 | **** |
| 3 | **** |
| 4 | **** |
| 5 | **** |
| 6 | **** |
| 7 | **** |
| 8 | **** |
| 9 | **** |
| 10 | *** |
| 11 | *** |
| 12 | *** |
| 13 | **** |
| 14 | **** |
| 15 | **** |

****: sehr gute Wirkung
*** : gute Wirkung
** : Wirkung
* : schwache Wirkung
0 : keine Wirkung

Verwendet man anstelle der erfindungsgemässen Formulierungen, die keinen Benzylalkohol und kein Spreitmittel enthalten, so wird ein Effekt, der dem der erfindungsgemässen Formulierungen entspricht, erst nach dreimaliger Applikation erreicht.

**Patentansprüche für die Vertragsstaaten:**
BE, CH, DE, FR, GB, IT, LI, NL, SE

1. Antimykotische Mittel in Emulsionsform mit höherer Freisetzung der Wirkstoffe, enthaltend Azolderivate und übliche Formulierungshilfsstoffe, dadurch gekennzeichnet, dass sie als Lösungsvermittler 3 bis 5 Gew.-% Benzylalkohol und als Spreitmittel 2,5 bis 20 Gew.-% 2-Octyldodecanol, Ölsäuredecylester, Isopropylmyristat, Isopropylpalmitat, Capryl/Caprinsäureester von gesättigten Fettalkoholen der Kettenlänge $C_{12}$-$C_{18}$, wachsartige Fettsäureester, Silikonöle, Isopropylmyristat/Isopropylstearat/Isopropylpalmitat-Gemisch oder Kokosfettsäureisopropylester enthalten.

2. Antimykotische Mittel nach Anspruch 1, dadurch gekennzeichnet, dass sie als Wirkstoff Clotrimazol der Formel

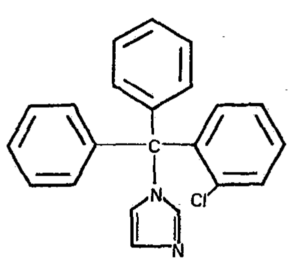

enthalten.

3. Antimykotische Mittel nach Anspruch 1, dadurch gekennzeichnet, dass sie als Wirkstoff Trifonazol der Formel

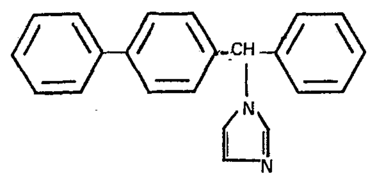

enthalten.

4. Antimykotische Mittel nach Anspruch 1, dadurch gekennzeichnet, dass sie als Wirkstoff Lombazol der Formel

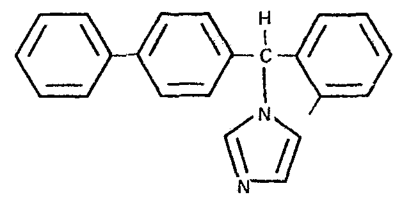

enthalten.

5. Antimykotische Mittel nach Anspruch 1, dadurch gekennzeichnet, dass sie die antimykotischen Azolderivate in Mengen von 0,05 Gew.-% bis 1 Gew.-%, vorzugsweise von 0,1 bis 1 Gew.-%, enthalten.

**Patentansprüche für den Vertragsstaat:** AT

1. Verfahren zur Herstellung von Antimykotika auf Basis von Azolderivatwirkstoffen und üblichen Formulierungshilfsstoffen, dadurch gekennzeichnet, dass die Wirkstoffe und Formulierungshilfsstoffe zusammen mit 3 bis 5% Masse Benzylalkohol als Lösungsvermittler und 2,5 bis 20% Masse 2-Octyldodecanol, Ölsäuredecylester, Isopropylmyristat, Isopropylpalmitat, Capryl/Caprinsäureester von gesättigten Fettalkoholen der Kettenlänge $C_{12}$-$C_{18}$, wachsartige Fettsäureester, Silikonöle, Isopropylmyristat/Isopropylstearat/Isopropylpalmitat-Gemisch oder Koksfettsäureisopropylester als Spreitmittel, jeweils bezogen auf die Gesamtmasse, als Emulsion formuliert werden.

2. Verfahren nach Anspruch 1, dadurch gekennzeichnet, dass die Wirkstoffe, insbesondere Clotrimazol, Trifonazol oder Lombazol, in Mengen von 0,05 bis 1% Masse, vorzugsweise von 0,1 bis 1% Masse, bezogen auf die Gesamtmasse, eingesetzt werden.

**Revendications pour les Etats Contractants:**
BE, CH, DE, FR, GB, IT, LI, NL, SE

1. Agents antimycotiques sous forme d'émulsion

à libération élevée des matières actives, contenant des dérivés azoliques et des auxiliaires de formulation usuels, caractérisés en ce qu'ils contiennent en tant que promoteur de dissolution 3 à 5% en poids d'alcool benzylique et comme agent d'étalement 2,5 à 20% en poids de 2-octyldodécanol, d'oléate de décyle, de myristate d'isopropyle, de palmitate d'isopropyle, d'ester caprylique/caprique d'alcools gras saturés de longueur de chaîne en $C_{12}$-$C_{18}$, d'esters d'acides gras cireux, d'huiles de silicones, de mélange myristate d'isopropyle/stéarate d'isopropyle/palmitate d'isopropyle ou d'ester isopropylique d'acides gras de coco.

2. Agents antimycotiques selon la revendication 1, caractérisés en ce qu'ils contiennent comme matière active du Clotrimazol de formule:

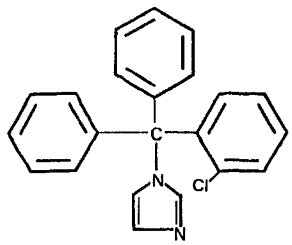

3. Agents antimycotiques selon la revendication 1, caractérisés en ce qu'ils contiennent comme matière active du Trifonazol de formule:

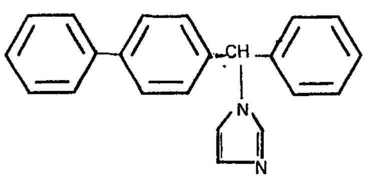

4. Agents antimycotiques selon la revendication 1, caractérisés en ce qu'ils contiennent comme matière active du Lombazol de formule:

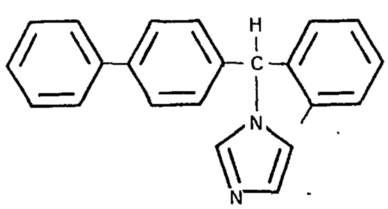

5. Agents antimycotiques selon la revendication 1, caractérisés en ce qu'ils contiennent les dérivés azoliques antimycotiques en des quantités de 0,05 à 1% en poids, de préférence de 0,1 à 1% en poids.

**Revendications pour l'Etat contractant:** AT

1. Procédé de préparation d'antimycotiques à base de matières actives de dérivés azoliques et d'auxiliaires de formulation usuels, caractérisé en ce qu'on formule sous la forme d'émulsion les matières actives et les auxiliaires de formulation conjointement avec 3 à 5% de la masse d'alcool benzylique comme promoteur de dissolution et avec 2,5 à 20% de la masse de 2-octyldodécanol, d'oléate de décyle, de myristate d'isopropyle, de palmitate d'isopropyle, d'ester caprylique/caprique d'alcools gras saturés de longueur de chaîne en $C_{12}$-$C_{18}$, d'esters d'acides gras cireux, d'huiles de silicones, de mélange myristate d'isopropyle/stéarate d'isopropyle/palmitate d'isopropyle ou d'ester isopropylique d'acide gras de coco en tant qu'agents d'étalement, dans chaque cas par rapport à la masse totale.

2. Procédé selon la revendication 1, caractérisé en ce que les matières actives, en particulier le Clotrimazol, le Trifonazol ou le Lombazol, sont utilisées en des quantités de 0,05 à 1% de la masse, de préference de 0,1 à 1% de la masse, par rapport à la masse totale.

**Claims for the Contracting States:**
BE, CH, DE, FR, GB, IT, LI, NL, SE

1. Antimycotic agents in emulsion form which have a higher degree of release of the active compounds and contain azole derivatives and customary formulation auxiliaries, characterised in that they contain as solubiliser 3 to 5% by weight of benzyl alcohol and as spreading agents 2.5 to 20% by weight of 2-octyldodecanol, oleic acid decyl ester, isopropyl myristate, isopropyl palmitate, caprylic/capric acid esters of saturated fatty alcohols of $C_{12}$-$C_{18}$ chain length, waxy fatty acid esters, silicone oils, and isopropyl myristate/isopropyl stearate/isopropyl palmitate mixture or coconut oil acid isopropyl ester.

2. Antimycotic agents according to Claim 1, characterised in that they contain clotrimazole of the formula

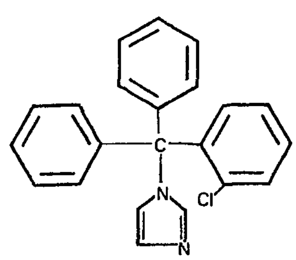

as the active compound.

3. Antimycotic agents according to Claim 1, characterised in that they contain trifonazole of the formula

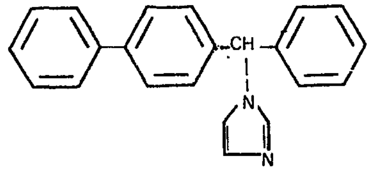

as the active compound.

4. Antimycotic agents according to Claim 1, characterised in that they contain lombazole of the formula

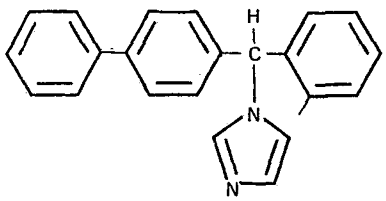

as the active compound.

5. Antimycotic agents according to Claim 1, characterised in that they contain the antimycotic azole derivatives in quantities of 0.05 to 1% by weight, preferably 0.1 to 1% by weight.

**Claims for the Contracting State:** AT

1: Process for the preparation of antimycotics based on azole derivative active compounds and customary formulation auxiliaries, characterised in that the active compounds and formulation auxiliaries are formulated as an emulsion together with 3 to 5% by weight of benzyl alcohol as the solubiliser and with 2.5 to 20% by weight of 2-octyldodecanol, oleic acid decyl ester, isopropyl myristate, isopropyl palmitate, caprylic/capric acid esters of saturated fatty alcohols of $C_{12}$-$C_{18}$ chain length, waxy fatty acid esters, silicone oils, an isopropyl myristate/isopropyl stearate/isopropyl palmitate mixture or coconut oil acid isopropyl ester as spreading agents, based in each case on the total weight.

2. Process according to Claim 1, characterised in that the active compounds, in particular clotrimazole, trifonazole or lombazole, are used in quantities of 0.05 to 1% by weight, preferably 0.1 to 1% by weight, based on the total weight.